# EUROPÄISCHE PATENTSCHRIFT

(11) **EP 4 081 122 B1**
(45) Veröffentlichungstag und Bekanntmachung des Hinweises auf die Patenterteilung: **09.04.2025**
(21) Anmeldenummer: 20839255.5
(22) Anmeldetag: 16.12.2020
(51) Int. Cl.: A61B 6/00, A61B 6/03

(54) **COMPUTERTOMOGRAPHIE-SYSTEM**
COMPUTER-ASSISTED TOMOGRAPHY SYSTEM
SYSTÈME DE TOMOGRAPHIE ASSISTÉE PAR ORDINATEUR

(30) Priorität: 23.12.2019 DE 102019135780
(43) Veröffentlichungstag der Anmeldung: 02.11.2022
(73) Patentinhaber: Otto-von-Guericke-Universität Magdeburg, 39106 Magdeburg (DE)
(72) Erfinder: ROSE, Georg, 39114 Magdeburg (DE); HOFFMANN, Thomas, 39112 Magdeburg (DE); LEOPOLD, Mathias, 39112 Magdeburg (DE); GROSSER, Oliver, 39108 Magdeburg (DE); PECH, Maciej, 39104 Magdeburg (DE)
(74) Vertreter: Günther, Constantin
(86) Internationale Anmeldenummer: PCT/EP2020/086486
(87) Internationale Veröffentlichungsnummer: WO 2021/130081

(56) Entgegenhaltungen:
- DE-A1- 102013 219 676
- DE-U1- 202015 106 190
- US-A1- 2011 096 894

## Beschreibung

Die Erfindung betrifft ein Computertomographie-System, nachfolgend auch CT-System genannt, für computertomographische Untersuchungen und Behandlungen an Patienten. Ein CT-System ist zum Beispiel aus der DE 10 2013 219 676 A1 bekannt. CT-Systeme können mit unterschiedlichen Arten von Röntgendetektoren betrieben werden. Eine Art von Röntgendetektor ist der sogenannte Mehrzeilendetektor, der den Vorteil einer Bildgebung mit einer hohen Dynamik (Kontrastauflösung) sowie eine sehr genaue Bestimmung der Hounsfield-Werte und sehr kurze Integrationszeiten durch die genutzten Szintillatormaterialien bietet. Es werden auch Flachdetektorsysteme als Röntgendetektor eingesetzt. Deren Vorteil ist eine sehr hohe räumliche Auflösung sowie ein großer Abbildungsbereich.

Aus der DE 20 2015 106 190 U1 ist eine Röntgendiagnostikvorrichtung zur Erfassung von CT-Daten und Projektionsdaten bekannt. Aus der US 2011/096894 A1 ist eine Röntgeneinrichtung mit einer Röntgenstrahlquelle und einem ersten und einem zweiten Röntgendetektor bekannt.

Der Erfindung liegt die Aufgabe zugrunde, ein solches CT-System hinsichtlich der Funktionalität weiter zu verbessern.

Diese Aufgabe wird gelöst durch ein CT-System mit folgenden Merkmalen:
a) wenigstens eine Röntgenstrahlquelle,
b) wenigstens einen Patiententisch zur Lagerung eines Patienten,
c) wenigstens einen ersten Röntgendetektor, der permanent oder zumindest zeitweise im Strahlengang der Röntgenstrahlen von der Röntgenstrahlquelle durch den Patienten angeordnet ist,
d) wenigstens einen zweiten Röntgendetektor, der permanent oder zumindest zeitweise im Strahlengang der Röntgenstrahlen von der Röntgenstrahlquelle durch den Patienten angeordnet ist,
e) wenigstens einen, z.B. automatisch, betätigbaren Antriebsmechanismus, durch den der erste und/oder der zweite Röntgendetektor von einer Position im Strahlengang der Röntgenstrahlen von der Röntgenstrahlquelle durch den Patienten zu einer Position außerhalb des Strahlengangs und umgekehrt bewegbar ist,
f) wenigstens eine elektronische Steuereinrichtung, die zur automatischen Betätigung des Antriebsmechanismus eingerichtet ist,
dadurch gekennzeichnet, dass der zweite Röntgendetektor in den Patiententisch baulich integriert ist, wobei der zweite Röntgendetektor als abgewinkelter CT-Flachdetektor oder als wenigstens zwei winklig zueinander angeordnete CT-Flachdetektoren ausgebildet ist.

Durch die Kombination aus erstem Röntgendetektor und zweitem Röntgendetektor kann die CT-Bildgebung wesentlich verbessert werden. Es können insbesondere unterschiedliche Wirkprinzipien für den ersten und den zweiten Röntgendetektor genutzt werden, wie zum Beispiel derart, dass der eine Röntgendetektor als Mehrzeilendetektor und der andere Röntgendetektor als Flachdetektor ausgebildet ist. Auf diese Weise können für die CT-Bildgebung die vorteilhaften Eigenschaften beider Wirkprinzipien genutzt werden.

Zusätzlich ist wenigstens ein z.B. automatisch betätigbarer Antriebsmechanismus vorhanden, durch den zumindest einer der Röntgendetektoren bewegt werden kann und auf diese Weise je nach Untersuchungs- und Behandlungssituation in den Strahlengang der Röntgenstrahlen bewegt oder außerhalb des Strahlengangs platziert werden kann. Hierbei wird davon ausgegangen, dass der Strahlengang der Röntgenstrahlen ausgehend von der Röntgenstrahlquelle durch den Patienten verläuft, der auf dem Patiententisch gelagert ist. Durch den Antriebsmechanismus kann der jeweilige bewegbare Röntgendetektor automatisiert an die gewünschte Position gebracht werden. Auf diese Weise sind keine manuellen Eingriffe erforderlich. Die elektronische Steuereinrichtung kann je nach Situation und Bedarf den Röntgendetektor mittels des automatisch betätigbaren Antriebsmechanismus entsprechend positionieren.

Die elektronische Steuereinrichtung kann z.B. einen Rechner aufweisen, der bestimmte Steuerungs- und/oder Regelungsschritte ausführt, z.B. mittels eines Computerprogramms. Der Rechner kann als handelsüblicher Computer ausgebildet sein, z.B. als PC, Laptop, Notebook, Tablet oder Smartphone, oder als Mikroprozessor, Mikrocontroller oder FPGA, oder als Kombination aus solchen Elementen.

Gemäß einer vorteilhaften Ausgestaltung der Erfindung ist vorgesehen, dass der erste Röntgendetektor in den Patiententisch baulich integriert ist oder der erste und/oder der zweite Röntgendetektor auf der von der Röntgenstrahlquelle aus gesehen abgewandten Seite des Patiententischs angeordnet ist oder durch den Antriebsmechanismus dort anordenbar ist. Der erste und/oder der zweite Röntgendetektor sind somit permanente Bestandteile des CT-Systems. Durch eine bauliche Integration eines oder beider Röntgendetektoren in den Patiententisch oder die Anordnung unter dem Patiententisch wird Platz gespart, sodass das erfindungsgemäße CT-System relativ kompakt ausgebildet sein kann. Zudem kann der automatisch betätigbare Antriebsmechanismus zumindest teilweise ebenfalls in den Patiententisch baulich integriert sein oder unter dem Patiententisch angeordnet sein.

Gemäß einer vorteilhaften Ausgestaltung der Erfindung kann der erste und/oder der zweite Röntgendetektor auch unabhängig vom Patiententisch sein. Beispielsweise kann das CT-System eine separate Detektoreinheit aufweisen, in die der erste und/oder der zweite Röntgendetektor baulich integriert ist. Der erste und/oder der zweite Röntgendetektor kann auch in einem Gehäuse des CT-Systems baulich integriert sein, beispielsweise rückseitig der Gantry. Beispielsweise kann der erste und/oder der zweite Röntgendetektor mittels des betätigbaren Antriebsmechanismus bei Bedarf in die Öffnung des CT-Systems hineinbewegt oder wieder herausbewegt werden.

Gemäß einer vorteilhaften Ausgestaltung der Erfindung ist vorgesehen, dass der erste Röntgendetektor als CT-Mehrzeilendetektor ausgebildet ist. Dies erlaubt eine besonders hochwertige Bildgebung der einzelnen CT-Bildschichten mit hohem Weichteilkontrast und hoher Geschwindigkeit.

Gemäß der Erfindung ist vorgesehen, dass der zweite Röntgendetektor als CT-Flachdetektor ausgebildet ist. Dies erlaubt eine sehr hohe räumliche Auflösung und einen großen Abbildungsbereich bei der Projektions-Bildgebung.

Das CT-System kann zum Beispiel derart ausgebildet sein, dass der CT-Flachdetektor mittels des automatisch betätigbaren Antriebsmechanismus bewegbar ist. In diesem Fall kann der CT-Flachdetektor bei der konventionellen CT-Bildgebung, für die der CT-Mehrzeilendetektor genutzt wird, außerhalb des Strahlengangs platziert werden. In diesem Fall kann ungestört die konventionelle CT-Bildgebung zum Beispiel rotierend mittels des Mehrzeilendetektors und der Röntgenstrahlquelle (CT-Röntgenröhre) durchgeführt werden. Wird ein breiter Abbildungsbereich in Form einer Radiographie oder Fluoroskopie benötigt, kann mittels des Antriebsmechanismus automatisch gesteuert durch die Steuereinrichtung der CT-Flachdetektor in den Strahlengang der Röntgenstrahlquelle gefahren werden. Für die Bildgebung mittels des CT-Flachdetektors kann die Röntgenstrahlquelle des CT-Systems selbst genutzt werden. Um eine kontinuierliche, zum Beispiel fluoroskopische, Bildgebung zu ermöglichen, kann die Gantryrotation angehalten oder eine getriggerte Bildgebung aus einem fixen Winkel innerhalb der Rotation erfolgen.

Gemäß der Erfindung ist vorgesehen, dass der zweite Röntgendetektor als abgewinkelter CT-Flachdetektor oder als wenigstens zwei winklig zueinander angeordnete CT-Flachdetektoren ausgebildet ist. Dies ermöglicht die Bildgebung aus größeren seitlichen Winkeln. Die bei der Bildgebung entstehenden Verzerrungen der Anatomie des Patienten können rechnerisch unter Einbeziehung der Rotationsparameter korrigiert werden. Der Winkel beim abgewinkelten CT-Flachdetektor oder bei den winklig zueinander angeordneten CT-Flachdetektoren kann beispielsweise im Bereich von 130° bis 170° liegen. Die Winkelangaben beziehen sich auf ein Kreismaß von 360 Grad (360°).

Gemäß einer vorteilhaften Ausgestaltung der Erfindung ist vorgesehen, dass der Antriebsmechanismus als elektromechanischer, hydraulischer oder pneumatischer Antriebsmechanismus oder als Kombination solcher Antriebsmechanismen ausgebildet ist. Dies erlaubt eine einfache und kostengünstige Realisierung eines zuverlässigen Antriebsmechanismus. Der Antriebsmechanismus kann zum Beispiel einen Elektromotor zum Bewegen des jeweiligen Röntgendetektors aufweisen, oder einen hydraulisch oder pneumatisch angetriebenen Stellzylinder, oder eine Kombination aus mehreren solcher Elemente.

Gemäß einer vorteilhaften Ausgestaltung der Erfindung ist vorgesehen, dass die Steuereinrichtung zusätzlich zur Steuerung der Röntgenstrahlquelle sowie zur Auswertung der Signale des ersten und des zweiten Röntgendetektors eingerichtet ist. Auf diese Weise sind in der Steuereinrichtung alle notwendigen Daten verfügbar, sowohl hinsichtlich der Signale des ersten und des zweiten Röntgendetektors als auch hinsichtlich der Art der Bestrahlung durch die Röntgenstrahlquelle sowie der Position des automatisch betätigbaren Antriebsmechanismus. Hierdurch lassen sich zusätzliche Informationen miteinander kombinieren, was zu einer Vereinfachung der CT-Bildgebung und zu einer Erhöhung der Qualität der Bildgebung führt. Auf diese Weise kann das gesamte CT-System günstiger bereitgestellt werden als markterhältliche Hybridsysteme.

Gemäß einer vorteilhaften Ausgestaltung der Erfindung ist vorgesehen, dass die Steuereinrichtung dazu eingerichtet ist, die Bildrekonstruktion der Computertomographie mittels einer Kombination der Signale des ersten und des zweiten Röntgendetektors durchzuführen. Auf diese Weise können Bildinformationen aus beiden Röntgendetektoren in die Bildrekonstruktion mit einfließen, was insgesamt ein Absenken der zu applizierenden Röntgenstrahldosis am Patienten ermöglicht.

Gemäß einer vorteilhaften Ausgestaltung der Erfindung ist vorgesehen, dass das CT-System wenigstens eine Anzeigeeinheit zum Anzeigen von aus den Signalen des ersten und/oder des zweiten Röntgendetektors gewonnenen Bildern aufweist. Auf der Anzeigeeinheit, zum Beispiel einem Bildschirm, können somit die durch die Bildrekonstruktion der Computertomographie erzeugten CT-Bilder und Radiographien dargestellt werden.

Die Erfindung wird nachfolgend anhand von Ausführungsbeispielen und der Verwendung von Zeichnungen näher erläutert.

Es zeigen
- Figur 1, 2: einen Teil des CT-Systems in Seitenansicht mit unterschiedlich positioniertem zweiten Röntgendetektor und
- Figur 3: eine vereinfachte Frontalansicht im Gantry-Aufbau des CT-Systems und
- Figur 4: eine weitere Ausführungsform des CT-Systems in Frontalansicht und
- Figur 5: eine schematische Block-Darstellung des gesamten CT-Systems.

Das in den Figuren dargestellte CT-System weist eine Röntgenstrahlquelle 1, einen Kollimator 12, einen Patiententisch 10, einen ersten Röntgendetektor 5, einen zweiten Röntgendetektor 4, einen automatisch betätigbaren Antriebsmechanismus 9, eine CT-Gantry 11, eine elektronische Steuereinrichtung 6, 7 und eine Anzeigeeinheit 8 auf.

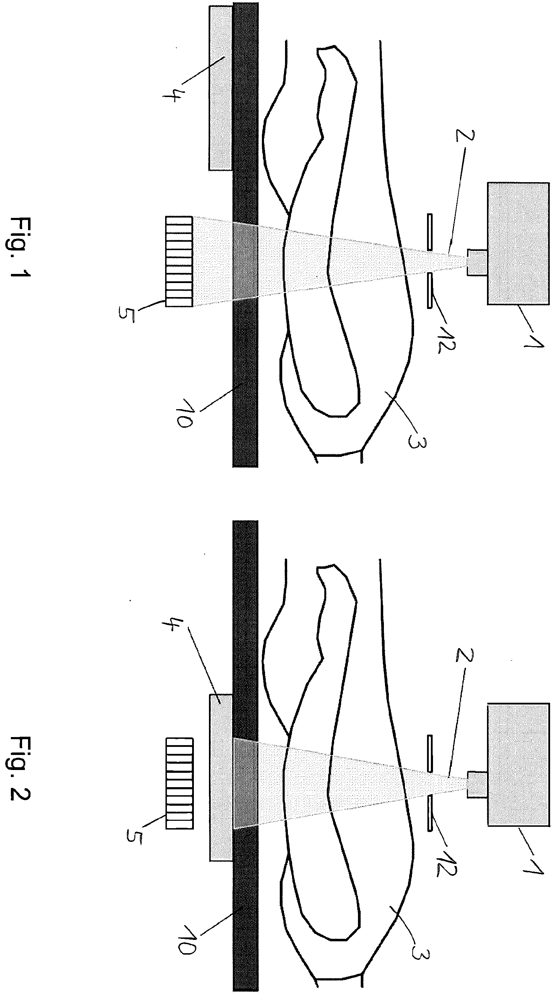

Wie man in Figur 1 erkennen kann, befindet sich ein Patient 3 auf dem Patiententisch 10 des CT-Systems. Die Röntgenstrahlquelle 1 gibt Röntgenstrahlen in einem Strahlengang 2 ab, die durch den Kollimator 12 geführt sind. Durch den Kollimator 12 erfolgt eine Einstellung des Strahlengangs. Auf der der Röntgenstrahlquelle 1 abgewandten Seite des Patienten 3, zum Beispiel unterhalb des Patiententischs 10, befindet sich der erste Röntgendetektor 5, zum Beispiel der übliche Mehrzeilendetektor. Zusätzlich ist der zweite Röntgendetektor 4, zum Beispiel ein Flachdetektor, vorhanden. Durch den in Figur 1 nicht erkennbaren Antriebsmechanismus 9 kann der zweite Röntgendetektor 4 von einer Position außerhalb des Strahlengangs 2, wie in Figur 1 dargestellt, in eine Position innerhalb des Strahlengangs 2 bewegt werden, beispielsweise einer Linear- oder Schwenk-Bewegung, wie die Figur 2 zeigt. In diesem Fall kann insbesondere eine Bildaufnahme mittels der Signale des zweiten Röntgendetektors 4 erfolgen. Der erste Röntgendetektor 5 kann in diesem Fall zumindest keine ausreichenden Signale für die Bildrekonstruktion liefern. Durch den Antriebsmechanismus 9 kann der zweite Röntgendetektor 4 wieder von der in Figur 2 dargestellten Position in die in Figur 1 dargestellte Position bewegt werden.

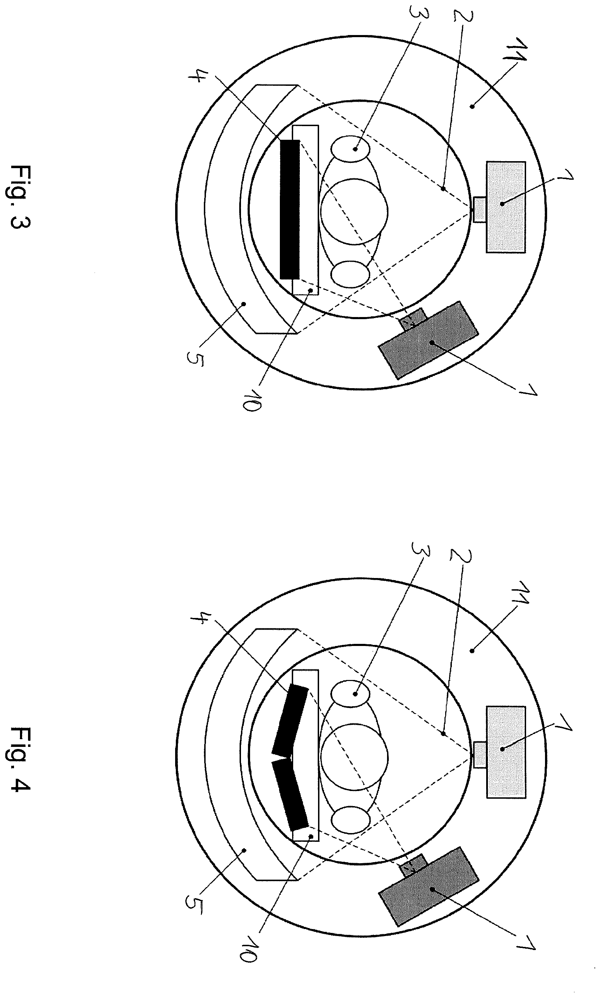

Die Figur 3 zeigt das CT-System gemäß Figur 2 mit im Strahlengang 2 befindlichem zweiten Röntgendetektor 4 in einer Frontaldarstellung durch die CT-Gantry 11. Dargestellt ist zudem, dass die Röntgenstrahlquelle 1, wie bei CT-Systemen üblich, zumindest in einem gewissen Bereich um den Patienten 3 herum rotiert werden kann, um eine CT-Bildrekonstruktion aus unterschiedlichen Bestrahlungswinkeln zu ermöglichen. Die Figur 3 zeigt dabei die Ausführung des zweiten Röntgendetektors 4 als Flachdetektor mit einer ebenen durchgehenden Detektorfläche.

Die Figur 4 zeigt eine gegenüber der Figur 3 insofern veränderte Ausführungsform, ebenfalls in der gleichen Frontaldarstellung, bei der der zweite Röntgendetektor 4 als abgewinkelter CT-Flachdetektor oder als Anordnung aus zwei winklig zueinander angeordneten CT-Flachdetektoren ausgebildet ist. Auf diese Weise kann ein größerer Winkelbereich bei der Signalerfassung durch den zweiten Röntgendetektor 4 abgedeckt werden.

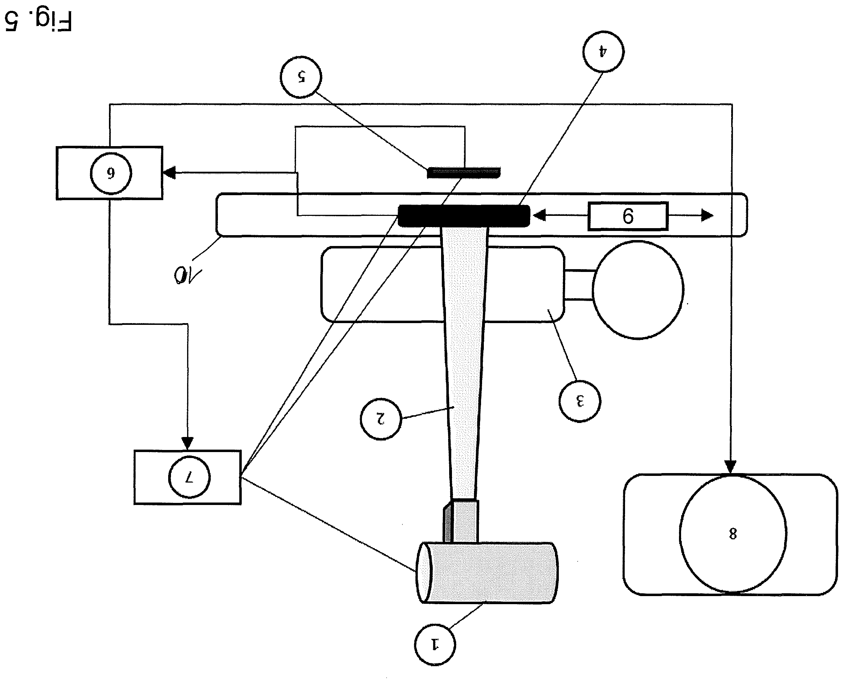

Die Figur 5 zeigt schematisch das gesamte CT-System. Erkennbar ist wiederum die Röntgenstrahlquelle 1 mit dem Strahlengang 2 der Röntgenstrahlen, der durch den Patienten 3 zu dem ersten Röntgendetektor 5 oder, wenn der zweite Röntgendetektor 4 in den Strahlengang bewegt ist, auch zum zweiten Röntgendetektor 4 verläuft.

Der Patient 3 ist wiederum auf dem Patiententisch 10 gelagert. Beispielsweise kann der zweite Röntgendetektor 4 in den Patiententisch 10 integriert sein.

Erkennbar ist zudem der automatisch betätigbare Antriebsmechanismus 9, durch den der zweite Röntgendetektor 4 in der beschriebenen Weise bewegt werden kann. Der Antriebsmechanismus 9 kann ebenfalls in den Patiententisch integriert sein.

Dargestellt ist ferner die Signalverbindung zwischen den bereits erwähnten Elementen des CT-Systems und der elektronischen Steuereinrichtung 6, 7, die beispielsweise eine Recheneinheit 6 und eine Steuerung 7 aufweisen kann. Über die Steuerung 7 kann eine Steuerung der Röntgenstrahlquelle 1, des ersten und des zweiten Röntgendetektors 4, 5 sowie des Antriebsmechanismus 9 erfolgen. Die Steuerung 7 ist mit der Recheneinheit 6 gekoppelt. Hierdurch kann die Recheneinheit 6 sämtliche Daten, die von dem ersten und dem zweiten Röntgendetektor 4, 5 geliefert werden, in Kombination mit den Steuerdaten der Steuerung 7 auswerten und insbesondere eine Bildberechnung mittels einer Kombination der Signale des ersten oder zweiten Röntgendetektors 4, 5 durchführen.

Die mittels der Recheneinheit 6 ermittelten CT-Bilder können auf der mit der Recheneinheit 6 gekoppelten Anzeigeeinheit 8 dargestellt werden.

## Patentansprüche

1. Computertomographie (CT)-System mit folgenden Merkmalen:
a) wenigstens eine Röntgenstrahlquelle (1),
b) wenigstens einen Patiententisch (10) zur Lagerung eines Patienten (3),
c) wenigstens einen ersten Röntgendetektor (5), der permanent oder zumindest zeitweise im Strahlengang (2) der Röntgenstrahlen von der Röntgenstrahlquelle (1) durch den Patiententisch (10) angeordnet ist,
d) wenigstens einen zweiten Röntgendetektor (4), der permanent oder zumindest zeitweise im Strahlengang (2) der Röntgenstrahlen von der Röntgenstrahlquelle (1) durch den Patiententisch (10) angeordnet ist,
e) wenigstens einen betätigbaren Antriebsmechanismus (9), durch den der erste und/oder der zweite Röntgendetektor (4, 5) von einer Position im Strahlengang (2) der Röntgenstrahlen von der Röntgenstrahlquelle (1) durch den Patiententisch (10) zu einer Position außerhalb des Strahlengangs (2) und umgekehrt bewegbar ist,
f) wenigstens eine elektronische Steuereinrichtung (6, 7), die zur Betätigung des Antriebsmechanismus (9) eingerichtet ist,
**dadurch gekennzeichnet, dass** der zweite Röntgendetektor (4) in den Patiententisch (10) baulich integriert ist, wobei der zweite Röntgendetektor (4) als abgewinkelter CT-Flachdetektor oder als wenigstens zwei winklig zueinander angeordnete CT-Flachdetektoren ausgebildet ist.

2. CT-System nach Anspruch 1, **dadurch gekennzeichnet, dass** der erste Röntgendetektor (5) in den Patiententisch (10) baulich integriert ist oder der erste und/oder der zweite Röntgendetektor (4, 5) von der Röntgenstrahlquelle (1) aus gesehen abgewandten Seite des Patiententischs (10) angeordnet ist oder durch den Antriebsmechanismus (9) dort anordenbar ist.

3. CT-System nach einem der vorhergehenden Ansprüche, **dadurch gekennzeichnet, dass** der erste Röntgendetektor (5) als CT-Mehrzeilendetektor ausgebildet ist.

4. CT-System nach einem der vorhergehenden Ansprüche, **dadurch gekennzeichnet, dass** der Antriebsmechanismus (9) als elektromechanischer, hydraulischer oder pneumatischer Antriebsmechanismus oder als Kombination solcher Antriebsmechanismen ausgebildet ist.

5. CT-System nach einem der vorhergehenden Ansprüche, **dadurch gekennzeichnet, dass** die Steuerungseinrichtung (6, 7) zusätzlich zur Steuerung der Röntgenstrahlquelle (1) sowie zur Auswertung der Signale des ersten und des zweiten Röntgendetektors (4, 5) eingerichtet ist.

6. CT-System nach Anspruch 5, **dadurch gekennzeichnet, dass** die Steuereinrichtung (6, 7) dazu eingerichtet ist, die Bildrekonstruktion der Computertomographie mittels einer Kombination der Signale des ersten und des zweiten Röntgendetektors (4, 5) durchzuführen.

7. CT-System nach einem der vorhergehenden Ansprüche, **dadurch gekennzeichnet, dass** das CT-System wenigstens eine Anzeigeeinheit (8) zum Anzeigen von aus den Signalen des ersten und des zweiten Röntgendetektors (4, 5) gewonnenen CT-Bildern aufweist.

## Claims

1. Computed tomography (CT) system with the following features:
a) at least one X-ray source (1),
b) at least one patient table (10) for positioning a patient (3),
c) at least one first X-ray detector (5), which is arranged permanently or at least temporarily in the beam path (2) of the X-rays from the X-ray source (1) through the patient table (10),
d) at least one second X-ray detector (4), which is arranged permanently or at least temporarily in the beam path (2) of the X-rays from the X-ray source (1) through the patient table (10),
e) at least one actuatable drive mechanism (9) by means of which the first and/or the second X-ray detector (4, 5) can be moved from a position in the beam path (2) of the X-rays from the X-ray source (1) through the patient table (10) to a position outside the beam path (2) and vice versa,
f) at least one electronic control device (6, 7) which is set up to actuate the drive mechanism (9),
**characterized in that** the second X-ray detector (4) is structurally integrated into the patient table (10), the second X-ray detector (4) being designed as an angled CT flat-panel detector or as at least two CT flat-panel detectors arranged at an angle to one another.

2. CT system according to claim 1, **characterized in that** the first X-ray detector (5) is structurally integrated into the patient table (10) or the first and/or the second X-ray detector (4, 5) is arranged on the side of the patient table (10) facing away from the X-ray source (1) or can be arranged there by the drive mechanism (9).

3. CT system according to one of the preceding claims, **characterized in that** the first X-ray detector (5) is designed as a CT multiline detector.

4. CT system according to one of the preceding claims, **characterized in that** the drive mechanism (9) is designed as an electromechanical, hydraulic or pneumatic drive mechanism or as a combination of such drive mechanisms.

5. CT system according to one of the preceding claims, **characterized in that** the control device (6, 7) is additionally set up to control the X-ray source (1) and to evaluate the signals of the first and second X-ray detectors (4, 5).

6. CT system according to claim 5, **characterized in that** the control device (6, 7) is set up to perform the image reconstruction of the computed tomography by means of a combination of the signals of the first and second X-ray detectors (4, 5).

7. CT system according to one of the preceding claims, **characterized in that** the CT system has at least one display unit (8) for displaying CT images obtained from the signals of the first and second X-ray detectors (4, 5).

## Revendications

1. Système de tomographie assistée par ordinateur (CT), comprenant les éléments suivants :
a) au moins une source de rayons X (1),
b) au moins une table de patient (10) pour le positionnement d'un patient (3),
c) au moins un premier détecteur de rayons X (5) disposé en permanence ou au moins temporairement dans le chemin optique (2) des rayons X depuis la source de rayons X (1) à travers la table de patient (10),
d) au moins un deuxième détecteur de rayons X (4) disposé en permanence ou au moins temporairement dans le chemin optique (2) des rayons X depuis la source de rayons X (1) à travers la table de patient (10),
e) au moins un mécanisme d'entraînement actionnable (9) permettant de déplacer le premier et/ou le deuxième détecteur de rayons X (4, 5) d'une position, située dans le chemin optique (2) des rayons X depuis la source de rayons X (1) à travers la table de patient (10), jusqu'à une position située en dehors du chemin optique (2), et inversement,
f) au moins un dispositif de commande électronique (6, 7) conçu pour actionner le mécanisme d'entraînement (9),
**caractérisé en ce que**
le deuxième détecteur de rayons X (4) est intégré structurellement dans la table de patient (10), le deuxième détecteur de rayons X (4) étant conçu comme un détecteur CT plat coudé ou comme au moins deux détecteurs CT plats disposés selon un angle l'un par rapport à l'autre.

2. Système CT selon la revendication 1,
**caractérisé en ce que** le premier détecteur de rayons X (5) est intégré structurellement dans la table de patient (10) ou le premier et/ou le deuxième détecteur de rayons X (4, 5) est disposé sur le côté de la table de patient (10) détourné de la source de rayons X (1) ou peut y être disposé par le mécanisme d'entraînement (9).

3. Système CT selon l'une des revendications précédentes,
**caractérisé en ce que** le premier détecteur de rayons X (5) est conçu comme un détecteur CT multiligne.

4. Système CT selon l'une des revendications précédentes,
**caractérisé en ce que** le mécanisme d'entraînement (9) est conçu comme un mécanisme d'entraînement électromécanique, hydraulique ou pneumatique ou comme une combinaison de tels mécanismes d'entraînement.

5. Système CT selon l'une des revendications précédentes,
**caractérisé en ce que** le dispositif de commande (6, 7) est en outre conçu pour commander la source de rayons X (1) et pour évaluer les signaux des premier et deuxième détecteurs de rayons X (4, 5).

6. Système CT selon la revendication 5,
**caractérisé en ce que** le dispositif de commande (6, 7) est conçu pour réaliser la reconstruction de l'image de la tomographie assistée par ordinateur au moyen d'une combinaison des signaux des premier et deuxième détecteurs de rayons X (4, 5).

7. Système CT selon l'une des revendications précédentes,
**caractérisé en ce que** le système CT comprend au moins une unité d'affichage (8) pour afficher les images CT obtenues à partir des signaux des premier et deuxième détecteurs de rayons X (4, 5).
